# FASCICULE DE BREVET EUROPEEN

(11) **EP 3 171 900 B1**
(45) Date de publication et mention de la délivrance du brevet: **01.04.2020**
(21) Numéro de dépôt: 15739560.9
(22) Date de dépôt: 16.07.2015
(51) Int. Cl.: A61L 2/12, A61L 11/00, B09B 3/00, H05B 6/78

(54) **SYSTEME DE TRAITEMENT EN CONTINU DE PRODUITS PAR APPORT THERMIQUE**
SYSTEM ZUR KONTINUIERLICHEN BEHANDLUNG VON PRODUKTEN DURCH WÄRMEEINBRINGUNG
SYSTEM FOR THE CONTINUOUS TREATMENT OF PRODUCTS BY THERMAL INPUT

(30) Priorité: 22.07.2014 FR 1457081
(43) Date de publication de la demande: 31.05.2017
(73) Titulaire: AMB, 7012 Mons (BE)
(72) Inventeur: HURLIN, Gauthier, 59125 Trith-St-Léger (FR); CORNEILLIE, Sébastien, 7600 Peruwelz (BE)
(74) Mandataire: Plasseraud IP
(86) Numéro de dépôt international: PCT/EP2015/066240
(87) Numéro de publication internationale: WO 2016/012334

(56) Documents cités:
- EP-A1- 0 710 636
- EP-A2- 0 103 396
- EP-A2- 0 334 460
- DE-C1- 3 505 570
- FR-A1- 2 646 083
- FR-A1- 2 909 079
- GB-A- 1 363 923
- US-A- 3 704 523
- US-A- 4 184 587
- US-A1- 2005 238 865

## Description

L'invention concerne un système de traitement en continu de produits par apport thermique. Un tel système trouve une application particulière, non limitative dans une installation d'hygiénisation ou de décontamination de déchets.

On entend par hygiéniser le fait de réduire à des taux acceptables les concentrations en agents pathogènes dans les déchets. A cet effet, on maintient les déchets dans une plage de température supérieure ou égale à 70°C, qui est typiquement comprise entre 70°C et 75°C.

On entend par décontaminer le fait de détruire les agents pathogènes dans les déchets à risque infectieux tels que les déchets médicaux. A cet effet, on maintient les déchets dans une plage de température supérieure ou égale à 100°C, typiquement comprise entre 100°C et 110°C.

On connaît de l'état de la technique des installations de traitement de déchets à risque infectieux par micro-ondes, fonctionnant en continu, permettant leur décontamination. Classiquement ce type d'installation de traitement en continu comprend un premier système assurant le déchiquetage des déchets, sous forme de granulats de taille suffisamment réduite au regard de la législation, un deuxième système de chauffage élevant rapidement les granulats à une température d'environ 100°C pour de la décontamination, et d'un troisième système maintenant, pendant un temps suffisant, les granulats à une température voisine mais supérieure de celle qu'ils ont atteint dans le deuxième système, de manière à assurer la décontamination des déchets.

Le document FR 2.646.083 décrit ainsi une installation de décontamination de déchets médicaux correspondant à la description générale énoncée au paragraphe précédent, dans lequel le deuxième système de chauffage met en œuvre une élévation de la température des déchets par micro-ondes dans l'enceinte d'un conduit recevant la vis d'un dispositif transporteur, ce dispositif transporteur assurant le déplacement du déchet entre le premier système et le troisième système.

Afin d'assurer la pénétration des micro-ondes dans le dispositif transporteur, il est nécessaire de limiter le diamètre du conduit formant l'enceinte du dispositif à une dizaine de centimètres. Ce faible diamètre provoque de fréquents blocages de la vis dans le conduit, ce qui entraîne des difficultés d'exploitation et des pertes de productivité.

La présente demanderesse a cherché à résoudre ce problème en proposant une installation (document FR 2.982.510) mettant en œuvre un traitement par micro-ondes dans une enceinte entourant un convoyeur vibrant. Une telle solution a pour inconvénient de nécessiter, pour confiner les vapeurs et les micro-ondes dans l'enceinte, la présence d'un joint dynamique entre une partie supérieure fixe et une partie inférieure mobile formant l'enceinte de traitement. En pratique, la mise en œuvre d'un tel joint dynamique s'avère complexe et coûteuse.

Le document DE 35 05 570 C1 fait partie du domaine de la décontamination des déchets infectieux, et en particulier des déchets hospitaliers et décrit une installation destinée à la décontamination de déchets infectieux. Les déchets infectieux arrivent par le convoyeur à bande et alimentent une trémie. Une fois la porte fermée, un dispositif est actionné et pulvérise sur les déchets de l'eau, puis les déchets sont déchiquetés par des rouleaux. Les déchets déchiquetés tombent sur la bande du convoyeur et sont transportés au travers d'une enceinte de traitement par micro-ondes.

Un carter en acier inoxydable définit les parois de l'enceinte micro-ondes à l'intérieur de laquelle les rayonnements sont contenus. Un second carter, plastique et interne au carter métallique, présente pour fonction de contenir les déchets dans un volume réduit de l'enceinte à parois métalliques. Des émetteurs micro-ondes sont pourvus extérieurement au carter plastique. Des capteurs infrarouges sont solidaires du couvercle du carter plastique, saillant intérieurement dans le volume intérieur du carter plastique afin de contrôler la température des produits dans l'enceinte.

Selon les constatations de l'inventeur, un premier défaut majeur de l'installation de ce document a pour origine l'utilisation d'un convoyeur à bande pour le transport des produits au travers de l'enceinte de traitement micro-ondes, en ce que les déchets peuvent comprendre des produits collants à la surface de la bande du convoyeur. Dans un tel cas, les produits adhérant à la bande passent continuellement du dessus vers le dessous du convoyeur lors de l'avance, sans jamais être évacués, et même lorsque le convoyeur est associé à une lèvre de raclage au niveau de la sortie, sensée décoller les produits de la bande, mais qui, en pratique, s'avère insuffisante.

C'est la raison pour laquelle, et dans ce domaine, il est classique pour l'homme du métier d'utiliser des convoyeurs à vis qui ne présentent pas ce défaut et tel qu'enseigné par le document FR 2.646.083. C'est encore la raison pour laquelle, et dans l'installation décrite dans le document FR 2.982.510 du présent demandeur, une solution à convoyeur vibrant a été choisie dans l'objectif de répondre aux deux problèmes identifiés ci-dessus, respectivement lorsque le convoyeur est à vis ou à bande.

Dans le dispositif de traitement continu par micro-ondes du document FR 2.646.083 à convoyeur à vis, celui du document DE 35 05 570C1 à convoyeur à bande, ou encore celui du document FR 2.982.510 à convoyeur vibrant, le confinement des déchet peut certes être amélioré par la présence d'un carter plastique, interne, destiné à confiner les déchets, dans un volume réduit d'une enceinte métallique dont la fonction est de contenir les micro-ondes. C'est ainsi que le document DE 35 05 570 C1 enseigne un carter plastique, interne à un carter métallique, dont les parois définissent l'enceinte micro-ondes. La fonction intrinsèque de ce carter plastique, repéré 15a, interne est de confiner les déchets et les vapeurs qui s'en dégagent dans un volume réduit de l'enceinte micro-ondes.

. Toutefois, et selon les constatations de l'inventeur, le confinement des déchets n'est pas optimal en ce qu'il existe nécessairement un espace vide entre la surface supérieure de la couche de produits à traiter, et le couvercle du carter plastique. Un tel espace vide est par exemple, visible à la figure 1 du document DE 35 05 570. Selon les constatations de l'inventeur, le couvercle plastique du carter de confinement est positionné par l'homme du métier à distance de la surface supérieure de la couche de déchets, afin d'éviter la présence de frottements entre la couche de produits et cette paroi de couvercle statique qui gêneraient la progression des déchets sous l'action du convoyeur, et seraient ainsi à l'origine de bourrage.

On connaît encore du domaine de la fabrication d'articles en céramique des installations de chauffage par traitement micro-ondes. Le document GB 1.363.923, ou encore le document US 3.704.523 enseignent de telles installations trouvant une application particulière pour le séchage des produits céramique formés.

Dans les deux cas, l'installation comprend un convoyeur à bande, assurant le transport de produits dans une chambre de traitement micro-ondes. Dans les deux cas, le traitement micro-ondes ne commence qu'après abaissement d'une structure d'enceinte, et jusqu'à sa mise en contact avec le convoyeur. Ainsi et dans le document US3.704.523, la structure d'enceinte repérée 11 est actionnée par le vérin repéré 6a, jusqu'à mise en contact de la base de la structure d'enceinte, repérée 18 avec la bande du convoyeur. Dans le document GB 1.363.923 la structure d'enceinte est repérée 15 et est actionnée par le vérin repéré 11a jusqu'à mise en contact de la base de la structure d'enceinte, repérée 20, avec la bande du convoyeur. Dans les deux cas, l'objectif poursuivi est de former une enceinte de traitement étanche pour les produits céramiques, avant la mise en œuvre du traitement micro-ondes qui s'effectue alors nécessairement lorsque le convoyeur à bande est à l'arrêt.

De telles installations de traitement enseignées par les documents US 3.704.523 ou GB 1.363.923 sont des installations de traitement discontinu en ce que le traitement micro-ondes n'est activé qu'après abaissement de la structure d'enceinte et donc l'arrêt du convoyeur, et contrairement aux systèmes de traitement par micro-ondes des documents DE 35 05 570 C1, FR 2.982.510 ou FR 2.646.083 qui assurent un traitement continu des produits dans l'enceinte de traitement, c'est-à-dire un traitement par micro-ondes des produits alors que ceux-ci sont déplacés dans l'enceinte par le convoyeur.

Le but de la présente invention est de pallier les inconvénients précités en proposant un système de traitement en continu par micro-ondes, de conception simplifiée, peu sensible au phénomène de blocage tel que rencontré dans les systèmes de traitement à convoyeur à vis, et peu sensible au problème des produits collants et mal évacués, tel que rencontré dans les convoyeurs à bande.

Un autre but de la présente invention est de proposer, au moins selon un mode de réalisation, un système de traitement de performance améliorée, en particulier, et selon les constatations des inventeurs, par un meilleur confinement des produits.

D'autres objectifs et avantages apparaîtront au cours de la description qui va suivre qui n'est donnée qu'à titre indicatif et qui n'a pas pour but de la limiter.

Un tel système trouvera une application particulière, non limitative, en tant que deuxième système et/ ou troisième système d'une installation de traitement de produits, dont l'architecture générale est décrite au paragraphe [004].

Aussi l'invention concerne tout d'abord un système de traitement en continu de déchets par apport thermique, pour l'hygiénisation des déchets ou la décontamination de tels déchets à risques infectieux comprenant :
- une enceinte de traitement des déchets,
- un dispositif d'apport thermique par générateurs de micro-ondes, agencé par rapport à ladite enceinte de manière à ce que les micro-ondes soient contenues dans ladite enceinte,
- un dispositif de transport par plancher mouvant, reposant sur le fond de l'enceinte, apte à assurer le transport d'une couche de déchets depuis leur entrée dans l'enceinte jusqu'à leur sortie de l'enceinte, comprenant un ensemble de lattes disposées en parallèle formant ledit plancher, ainsi qu'un système de commande des lattes selon un mouvement alternatif, apte à assurer le transport d'une couche de déchets
- un dispositif de confinement des déchets et des vapeurs qui s'en dégagent, interne à ladite enceinte.

Selon des caractéristiques optionnelles de l'invention, prises seules ou en combinaison :
- ledit dispositif de confinement de déchets et des vapeurs qui s'en dégagent comprend une plaque de confinement, interne à ladite enceinte, destinée à appuyer sur la couche de produits, mobile par rapport à une partie fixe de l'installation ;
- la plaque de confinement est disposée dans ladite enceinte entre d'une part les générateurs micro-ondes dudit dispositif d'apport thermique par générateurs de micro-ondes et d'autre part la couche de produits, ladite plaque de confinement étant dans une matière perméable aux micro-ondes telle que le polytetrafluoroéthylène ;
- ladite plaque de confinement est suspendue à une partie fixe de l'installation par un système de balanciers ;
- le dispositif de transport par plancher mouvant est formé d'un ensemble de lattes constituées d'au moins trois sous-ensembles, lesdites lattes étant disposées en parallèle, ainsi qu'un système de commande séquentielle des lattes selon un mouvement alternatif, et le cas échant le système comprend un dispositif de chauffage de l'ensemble de lattes de telle façon à constituer un plancher mouvant chauffant.

Selon une autre alternative, le dispositif de confinement des déchets et des vapeurs qui s'en dégagent comprend un tapis roulant, interne à ladite enceinte, ledit tapis roulant comprenant une bande souple, guidée par des cylindres de guidage, rotatifs, ladite bande souple étant destinée à appuyer sur la couche de déchets.

De préférence, le tapis roulant est disposé dans ladite enceinte entre d'une part les générateurs de micro-ondes dudit dispositif d'apport thermique par générateurs de micro-ondes et d'autre part la couche de déchets, ladite bande souple étant destinée à être traversée par les micro-ondes et étant réalisée dans une matière perméable aux micro-ondes telle que le silicone.

L'invention concerne encore une installation de traitement en continu de déchets comprenant, en série :
- un premier système de déchiquetage voire de mélange de déchets,
- un deuxième système de traitement en continu de déchets par apport thermique pour l'élévation en température des déchets,
- un troisième système pour le maintien en température des déchets.

Selon l'invention, le deuxième système ou le troisième système est constitué par un système de traitement en continu de produits, conforme à l'invention. Eventuellement, le deuxième système et le troisième système sont constitués chacun essentiellement par un système conforme à l'invention.

L'invention sera mieux comprise à la lecture de la description suivante accompagnée des dessins en annexe parmi lesquels :
- La figure 1 est une vue d'une installation conforme à l'invention selon un premier mode de réalisation, comprenant un système selon l'invention comme second système pour élever les produits en température, le troisième système pour maintenir les produits en température comprenant un dispositif de transport du type à vis sans fin.
- La figure 2 est une vue d'une installation conforme à l'invention, selon un second mode de réalisation, comprenant un système selon l'invention comme second système pour élever les produits en température et un autre système conforme à l'invention comme troisième système pour maintenir les produits en température,
- La figure 3 est une vue d'une installation conforme à l'invention, selon un troisième mode de réalisation, comprenant un système selon l'invention comme second système pour élever les produits en température et un troisième système pour maintenir les produits en température,
- La figure 4 est une vue, selon une coupe verticale, d'un système conforme à l'invention,
- Les figures 5a à 5e illustrent les différentes étapes d'un cycle d'avancement du dispositif de transport à plancher mouvant,
- La figure 6 est une vue de détail, de coupe, de trois lattes consécutives d'un plancher mouvant qui peut être mis en œuvre selon l'invention,
- La figure 7 est une vue d'une installation selon la figure 3 dans laquelle le dispositif de confinement du type à plaque de confinement suspendue est remplacé par un dispositif de confinement du type à tapis roulant.

Aussi l'invention concerne un système de traitement en continu de produits par apport thermique comprenant :
- une enceinte de traitement 210 ; 220 ; 320 de produits,
- un dispositif 211 ; 221 ; 321 d'apport thermique par générateurs de micro-ondes 211a ; 221a ; 321a, agencé par rapport à ladite enceinte 210 ; 220 ; 320 de manière à ce que les micro-ondes soient contenues dans ladite enceinte,
- un dispositif de transport 212 ; 222 ; 322 par plancher mouvant, reposant sur le fond de l'enceinte 210 ; 220 ; 320, apte à assurer le transport d'une couche de produits D depuis leur entrée dans l'enceinte et jusqu'à leur sortie de l'enceinte,
- un dispositif 213 ; 223 ; 323 de confinement de produits et des vapeurs qui s'en dégagent, interne à ladite enceinte.

L'enceinte de traitement est définie entre un fond 210a ; 220a ; 320a, un couvercle 210b ; 220b ; 320b, et des parois latérales reliant le fond 210a ; 220a ; 320a au couvercle 210b ; 220b ; 320b. La paroi du fond, la paroi du couvercle et les parois latérales sont réalisées dans une matière imperméable aux micro-ondes afin de contenir les micro-ondes à l'intérieur de l'enceinte.

De préférence, le fond 210a ; 220a ; 320a, le couvercle 210b ; 220b ; 320b et les parois latérales forment un tunnel, notamment de section rectangulaire, s'étendant depuis l'entrée des produits dans l'enceinte jusqu'à leur sortie de l'enceinte.

Le dispositif 211 ; 221 ; 321 d'apport thermique comprend au moins un générateur de micro-ondes 211a ; 221a ; 321a. Ces générateurs micro-ondes sont situés en dehors de l'enceinte 210 ; 220 ; 320, et de préférence au-dessus du couvercle 210b ; 220b ; 320b. A cet effet, la paroi du couvercle présente, au droit des générateurs, des zones locales perméables aux micro-ondes.

Le dispositif de transport 212 ; 222 ; 322 par plancher mouvant, reposant sur le fond de l'enceinte 210 ; 220 ; 320, est apte à assurer le transport d'une couche de produits D, depuis leur entrée dans l'enceinte jusqu'à leur sortie. Un tel dispositif de transport, connu en tant que tel de l'état de la technique, comprend classiquement un ensemble de lattes disposées en parallèle et formant ledit plancher, ainsi qu'un système de commande des lattes selon un mouvement alternatif. Un tel dispositif de transport par plancher mouvant, encore usuellement dénommé «*fond mouvant»* n'est toutefois pas connu de l'état de la technique du domaine de l'invention, à savoir celui des dispositifs de traitement continu par micro-ondes, en particulier de ceux utilisés pour la décontamination ou l'hygiénisation des déchets, mais seulement du domaine particulier des semi-remorques. De tels fonds mouvants sont alors utilisés pour le déchargement de produits en vrac de la caisse du semi-remorque.

L'ensemble de lattes d'un tel plancher est formé typiquement d'au moins trois sous-ensembles de lattes, repérés respectivement 212a ; 212b ; 212c et comprenant chacun au moins N lattes, N étant un nombre entier positif. Les N lattes de chaque sous-ensemble peuvent être déplacées conjointement, indépendamment des N lattes des autres sous-ensembles. Chaque latte d'un sous-ensemble est séparée de la voisine du même sous-ensemble par au moins deux lattes appartenant chacune à l'un des autres sous-ensembles.

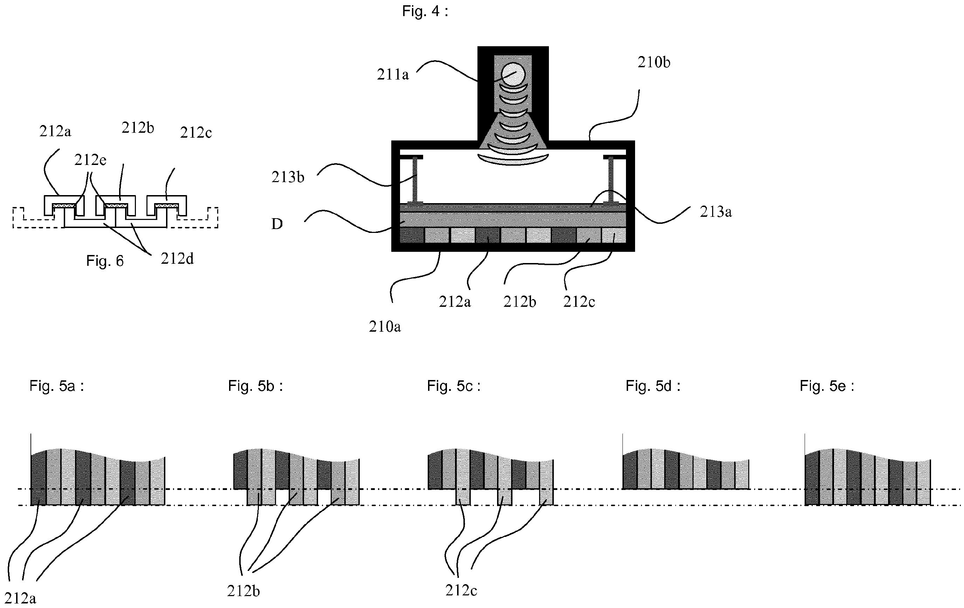

Le cycle d'avancement mis en œuvre par le système de commande pour un plancher mouvant comprenant, à titre d'exemple, trois sous-ensembles de lattes, ainsi que ses différentes étapes sont illustrés aux figures 5a à 5e.

A la figure 5a, les lattes des trois sous-ensembles 212a, 212b et 212c sont alignées. Le système de commande actionne, dans une première étape, les lattes du sous-ensemble 212a, au recul, jusqu'à la position illustrée à la figure 5b, alors que les lattes des deux autres sous-ensembles 212b et 212c restent fixes. Lors de cette action, les matières reposant sur le plancher ne bougent pas, les lattes du sous-ensemble 212a glissant sous les produits.

Le système de commande actionne, dans une deuxième étape, les lattes du sous-ensemble 212b au recul jusqu'à la position illustrée à la figure 5c alors que les lattes des deux autres sous-ensembles 212a et 212c restent fixes. Lors de cette action, les produits reposant sur le plancher ne bougent pas, les lattes du sous-ensemble 212b glissant sous les produits.

Le système de commande actionne, dans une troisième étape, les lattes du sous-ensemble 212c au recul jusqu'à la position illustrée à la figure 5d alors que les lattes des deux autres sous-ensembles 212a et 212b restent fixes. Lors de cette action, là encore, les produits reposant sur le plancher ne bougent pas, les lattes du sous-ensemble 212c glissant sous les produits.

Enfin, dans une quatrième étape, l'ensemble des lattes des différents sous-ensembles 212a, 212b, 212c sont actionnées simultanément à l'avance, provoquant l'avancement des produits d'un pas. Le cycle ainsi décrit en quatre étapes est répété pour faire avancer les déchets pas à pas.

Les lattes mobiles voisines, appartenant à chaque sous-ensemble de lattes 212a, 212b, 212c du plancher mouvant peuvent ne pas être jointives telles qu'illustrées à la figure 6, mais au contraire être écartées l'une de l'autre selon une configuration connue en tant que telle de l'état de la technique des planchers mouvants. En particulier, des éléments de guidage 212d, fixes, inférieurs, de section en U, peuvent assurer le guidage en translation des lattes appartenant à chaque sous-ensemble 212a, 212b, 212c elles-mêmes de section en U inversée et positionnées à cheval sur les extrémités de deux éléments de guidage 212d juxtaposés, par l'intermédiaire d'un joint 212e. Une telle configuration de plancher mouvant assure par ailleurs une bonne étanchéité au liquide.

Le dispositif 213 ; 223 ; 323 de confinement de produits et des vapeurs qui s'en dégagent est interne à ladite enceinte 210 ; 220 ; 320 et est perméable aux micro-ondes. Il est disposé entre la couche de produits D et les générateurs micro-ondes 211a ; 221a ; 321a. La fonction de ce dispositif est de contenir localement les produits et les vapeurs qui s'en dégagent, dans un volume réduit de ladite enceinte.

Un tel dispositif 213 ; 223 ; 323 de confinement permet avantageusement d'améliorer les performances thermiques dans l'enceinte de traitement, en limitant les pertes de chaleur entre la couche de produits et l'atmosphère présente dans l'enceinte. Un tel dispositif de confinement permet d'élever les produits à plus haute température ou à tout le moins d'augmenter le rendement énergétique du système.

Selon un mode de réalisation, le dispositif de confinement comprend une plaque de confinement 213a ; 323a, interne à ladite enceinte 210 ; 320 destinée à appuyer sur le dessus de la couche de produits D. Cette plaque de confinement est imperméable aux vapeurs, mais est réalisée dans un matériau perméable aux micro-ondes (i.e. le polytetrafluoroéthylène : PTFE), de manière à être traversé par ces dernières. Cette plaque de confinement 213a ; 323a est de préférence sensiblement parallèle au plancher mouvant et est de préférence mobile par rapport à une partie fixe de l'installation via des moyens de suspension tels que des balanciers 213b ; 323b.

Comme visible à la figure 5, cette plaque 213a ; 323a s'étend en largeur d'une paroi latérale à l'autre paroi latérale de l'enceinte, et en longueur, à proximité de l'entrée des produits dans l'enceinte et jusqu'à proximité de leur sortie de l'enceinte. La couche de produits D, ainsi que les vapeurs qui s'en dégagent sont alors contenues dans le dispositif de confinement entre le plancher, la sous-face de la plaque de confinement 213a ; 323a et les sections des parois latérales s'étendant entre la plaque de confinement et le plancher.

Les moyens de suspension de la plaque de confinement 213a ; 323a à ladite partie fixe peuvent être des balanciers 213b ; 323b ou des mécanismes équivalents, passifs, permettant à la plaque de confinement de reposer sous son propre poids sur la couche de produits D, tout en autorisant son soulèvement lors de l'avancement des produits, sous l'action du plancher mouvant. En appuyant sur le dessus de la couche de produits de préférence sous l'action de son propre poids, la plaque de confinement permet avantageusement de maintenir une épaisseur uniforme de la couche de produits.

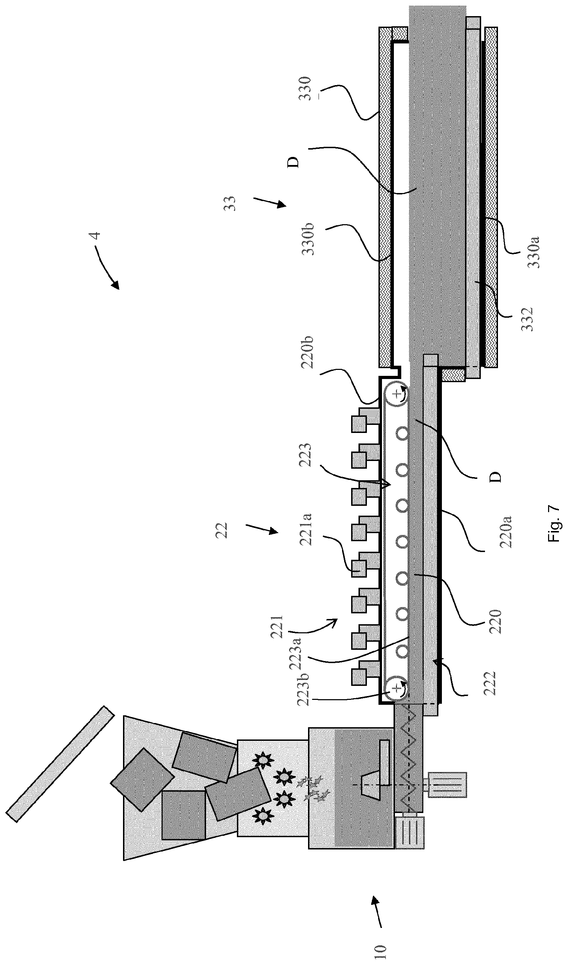

Alternativement, et selon un mode de réalisation illustré à la figure 7, le dispositif 223 de confinement de produits et des vapeurs qui s'en dégagent comprend un tapis roulant, interne à la dite enceinte 220, ledit tapis roulant comprenant une bande souple 223a, guidée par des cylindres de guidage 223b, rotatifs, ladite bande souple 223a étant destinée à appuyer sur le dessus de la couche de produits D. Le tapis roulant est disposé dans ladite enceinte 220 entre d'une part les générateurs de micro-ondes 221a dudit dispositif 221 d'apport thermique par générateurs de micro-ondes et d'autre part la couche de produits D, ladite bande souple 223a étant destinée à être traversée par les micro-ondes et étant réalisée dans une matière perméable aux micro-ondes telle que le silicone.

La fonction de ce tapis roulant est de confiner les produits et les vapeurs dans un volume défini entre le tapis roulant, et les sections des parois latérales entre le tapis et le plancher mouvant. Les cylindres de guidage 223b, libres en rotation, permettent à la bande souple 223a d'avancer, sous l'action de la couche de produits D, à chaque avance provoquée par le plancher mouvant et de manière à ne pas gêner la progression des produits.

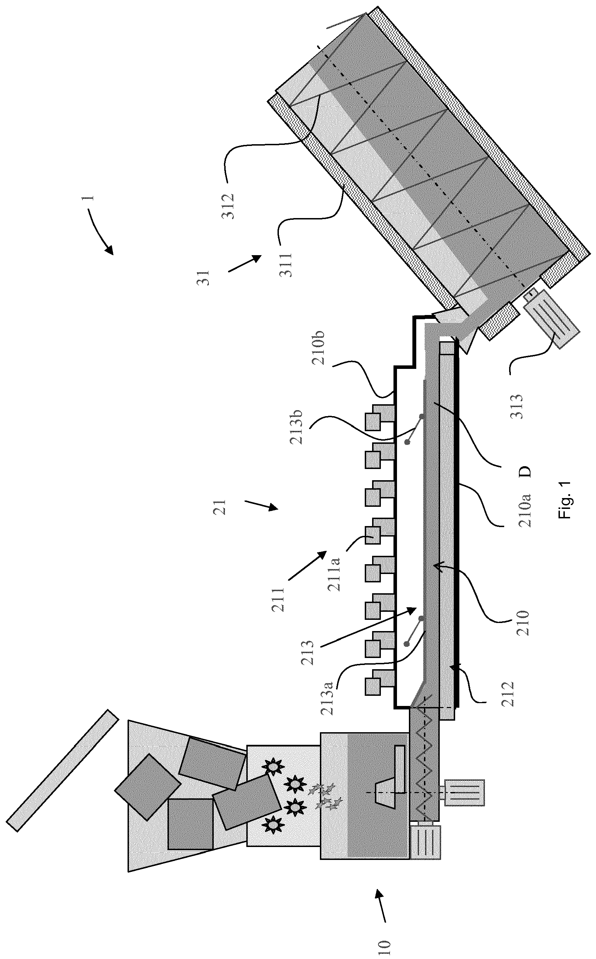

Dans les deux cas qu'il s'agisse du dispositif de confinement à plaque de confinement mobile, ou encore du dispositif de confinement à tapis roulant, le dispositif de confinement forme un couvercle physique en appui sur la couche de produits, avantageusement sans que ce couvercle ne gêne la progression des produits par l'action du plancher mouvant, et sans espace vide entre la couche de produit et le dispositif de confinement, et comme visible à la figure 1 du document DE 35 05 570C1.

Selon un mode de réalisation, le système peut permettre un apport thermique supplémentaire par le plancher mouvant qui peut être chauffé directement par tout moyen connu, notamment résistif.

L'invention concerne également une installation 1 ; 2 ; 3 ; 4 de traitement en continu des déchets comprenant, en série :
- un premier système 10 de déchiquetage voire de mélange de produits, et,
- un deuxième système 21 ; 22 de traitement en continu de produits par apport thermique, pour l'élévation en température des déchets,
- un troisième système 31 ; 32 ; 33 pour le maintien en température des produits.

Selon l'invention, le deuxième système ou le troisième système est constitué essentiellement par un système de traitement en continu de produits, conforme à l'invention. Eventuellement le deuxième système et le troisième système sont constitués chacun par un tel système conforme à l'invention.

Le premier système 10 peut comprendre une trémie, suivie de moyens pour déchiqueter les produits en granulats, voire de moyens de mélange des granulats. Le deuxième système 21 ; 22 est constitué essentiellement par un système conforme à l'invention.

L'entrée de l'enceinte de traitement 210 ; 220 du deuxième système 21 ; 22 peut être alimenté par un ou plusieurs convoyeurs à vis, de manière à former une couche de produits, dans l'enceinte 210 ; 220, notamment entre la plaque de confinement 213a et le dispositif de transport par plancher mouvant 212, tel qu'illustré aux figures 1 à 3, ou encore entre la bande souple 223a du tapis roulant et le dispositif de transport par plancher mouvant 222 tel qu'illustré à la figure 7.

Le système de commande du plancher mouvant permet le déplacement de la couche de produits depuis leur entrée dans l'enceinte jusqu'à leur sortie de l'enceinte à partir de laquelle les produits sont acheminés, de préférence par gravité, jusqu'à l'entrée du troisième système 31 ; 32 ; 33 destiné au maintien en température des déchets.

Dans l'enceinte de traitement 210 ; 220 du deuxième système, la température des produits est rapidement élevée par les micro-ondes, voire également par un apport thermique supplémentaire provenant du plancher mouvant. Dans le cas d'un traitement de décontamination de déchets à risque infectieux, le deuxième système permet d'élever la température des déchets au dessus de 100°C, de l'ordre de 100°C par exemple entre 100°C et 110°C. Dans le cas d'une hygiénisation des déchets, le deuxième système permet d'élever la température des déchets au dessus de 70°C, par exemple entre 70°C et 75°C.

Le troisième système 31 ; 32 ; 33 destiné au maintien en température des produits permet le maintien à une température voisine, mais supérieure de celle des produits sortant du deuxième système, par exemple de plus 5°C.

Ce troisième système peut présenter différentes formes. Selon un premier mode de réalisation, notamment illustré à la figure 1, ce troisième système peut comprendre une cuve chauffée 311, à l'intérieur de laquelle tourne une vis sans fin 312, qui assure le déplacement des produits dans ce troisième système 31. La vis sans fin 312 est entraînée par le moteur 313.

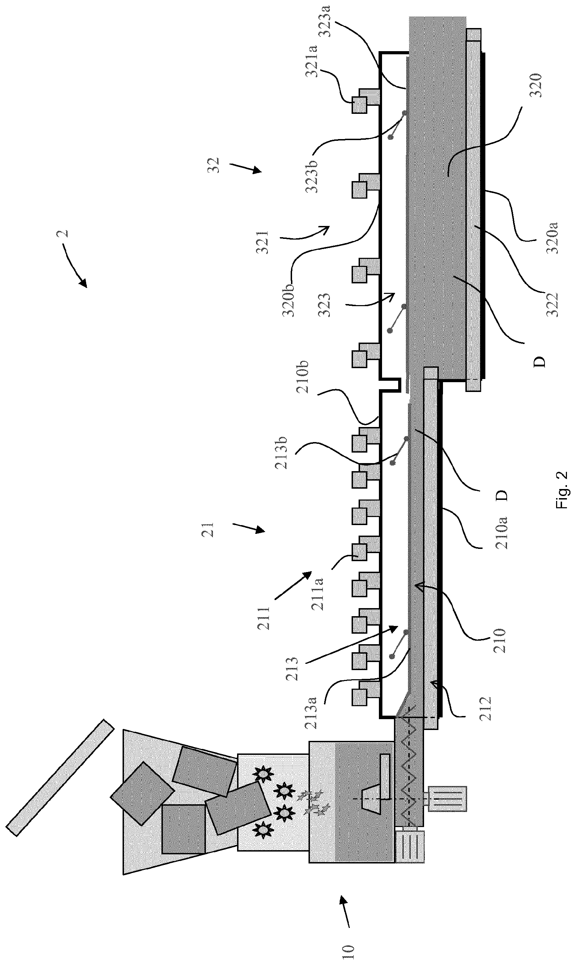

Selon un second mode de réalisation, notamment illustré à la figure 2, le troisième système peut être constitué essentiellement par un système conforme à l'invention, comprenant :
- une enceinte de traitement 320, formée entre un couvercle 320b, un fond 320a et des parois latérales,
- un dispositif 321 d'apport thermique par générateurs de micro-ondes 321a, les générateurs étant agencés extérieurs à l'enceinte, au-dessus du couvercle 320b de manière à ce que les micro-ondes soient contenues dans ladite enceinte,
- un dispositif de transport 322 par plancher mouvant, reposant sur le fond de l'enceinte 320, apte à assurer le transport d'une couche de produits D depuis leur entrée dans l'enceinte jusqu'à leur sortie de l'enceinte,
- un dispositif 323 de confinement de produits et des vapeurs qui s'en dégagent, interne à ladite enceinte, comprenant une plaque de confinement 323a, mobile, en appui sur les produits et suspendue par rapport à une partie fixe de l'installation, par un système de balanciers tels que 323b.

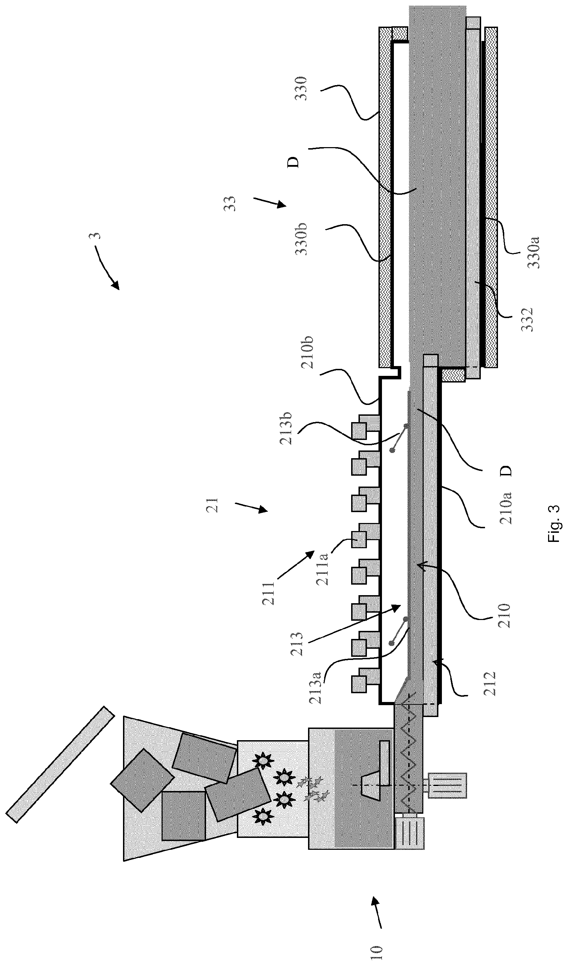

Selon un troisième mode de réalisation, notamment illustré à la figure 3 ou encore à la figure 7, le troisième système 33 comprend une enceinte 330 formée entre un couvercle 330b, un fond 330a, et des parois latérales formant un tunnel, ainsi qu'un plancher mouvant 332 reposant sur le fond 330a. Dans cette enceinte, les produits sont maintenus en température par chauffage des parois, par exemple au moyen d'un chauffage résistif.

### NOMENCLATURE

- 1, 2, 3, 4. Installation de traitement en continu de produits,
- 10. Premier système de déchiquetage voire de mélange de produits,
- 21, 22. Deuxième système de traitement en continu de produits par apport thermique pour l'élévation en température des produits,
- 31, 32, 33. Troisième système pour le maintien en température des produits,
- 210, 220, 320, 330 Enceinte de traitement de produits,
- 211, 221, 321. Dispositif d'apport thermique par générateurs de micro-ondes,
- 212, 222, 322, 332. Dispositif de transport par plancher mouvant,
- 213, 223, 323. Dispositif de confinement de produits et des vapeurs, interne à l'enceinte.
- 311. Cuve chauffée,
- 312. Vis sans fin,
- 313. Moteur,
- D. Couche de déchets
- 210a, 220a, 320a, 330a. Fond,
- 210b, 220b, 320b, 330b. Couvercle,
- 211a, 221a, 321a. Générateurs de micro-ondes,
- 212a. Premier sous-ensemble de lattes,
- 212b. Deuxième sous-ensemble de lattes,
- 212c. Troisième sous-ensemble de lattes,
- 212d. Éléments de guidage des lattes, de section en U,
- 212e. Joints d'étanchéité,
- 213a, 323a. Plaque de confinement,
- 213b, 323b. Système de balancier,
- 223a. Bande souple du tapis roulant,
- 223b. Cylindres de guidage du tapis roulant.

## Revendications

1. Système (21 ; 22 ; 32) de traitement en continu de déchets par apport thermique, pour l'hygiénisation des déchets ou la décontamination de tels déchets à risque infectieux comprenant :
- une enceinte de traitement (210 ; 220 ; 320) de déchets,
- un dispositif (211 ; 212 ; 321) d'apport thermique par générateurs de micro-ondes (211a ; 221a ; 321a), agencé par rapport à ladite enceinte (210 ; 220 ; 320) de manière à ce que les micro-ondes soient contenues dans ladite enceinte (210 ; 220 ; 320)
- un dispositif (213 ; 223 ; 323) de confinement des produits et des vapeurs qui s'en dégagent, interne à ladite enceinte,
- un dispositif de transport (212 ; 222 ; 322) par plancher mouvant, reposant sur le fond de l'enceinte (210 ; 220 ; 320), comprenant un ensemble de lattes disposées en parallèle formant ledit plancher, ainsi qu'un système de commande des lattes selon un mouvement alternatif, apte à assurer le transport d'une couche de déchets (D) depuis leur entrée dans l'enceinte jusqu'à leur sortie de l' enceinte.

2. Système selon la revendication 1, dans lequel ledit dispositif (213 ; 323) de confinement des déchets et des vapeurs qui s'en dégagent comprend une plaque de confinement (213a ; 323a), interne à ladite enceinte (210 ; 320), destinée à appuyer sur la couche de déchets (D), mobile par rapport à une partie fixe du système via des moyens de suspension, passifs, permettant à la plaque de confinement (213a ; 323a) de reposer sous son propre poids sur la couche de produits (D), tout en autorisant son soulèvement lors de l'avancement des produits sous l'action du dispositif de transport par plancher mouvant.

3. Système selon la revendication 2, dans lequel la plaque de confinement (213a ; 323a) est disposée dans ladite enceinte (210 ; 320) entre d'une part les générateurs de micro-ondes (211a ; 321a) dudit dispositif (211 ; 321) d'apport thermique par générateurs de micro-ondes et d'autre part la couche de déchets (D), ladite plaque de confinement (213a ; 323a) étant destinée à être traversée par les micro-ondes et étant réalisée dans une matière perméable aux micro-ondes telle que le polytetrafluoroéthylène.

4. Système selon la revendication 2 ou 3, dans lequel ladite plaque de confinement (213a ; 323a) est suspendue à la partie fixe de l'installation par un système de balanciers (213b ; 323b).

5. Système selon la revendication 1, dans lequel le dispositif (223) de confinement des déchets et des vapeurs qui s'en dégagent comprend un tapis roulant, interne à la dite enceinte (220), ledit tapis roulant comprenant une bande souple (223a), guidée par des cylindres de guidage (223b), rotatifs, ladite bande souple (223a) étant destinée à appuyer sur la couche de déchets (D), .

6. Système selon la revendication 5, dans lequel le tapis roulant est disposé dans ladite enceinte (220) entre d'une part les générateurs de micro-ondes (221a) dudit dispositif (221) d'apport thermique par générateurs de micro-ondes et d'autre part la couche de déchets (D), ladite bande souple (223a) étant destinée à être traversée par les micro-ondes et étant réalisée dans une matière perméable aux micro-ondes telle que le silicone.

7. Système selon l'une des revendications 1 à 6, dans lequel le dispositif de transport (212 ; 222 ; 322) par plancher mouvant est formé dudit ensemble de lattes constituées d'au moins trois sous-ensembles (212a, 212b, 212c), lesdites lattes étant disposées en parallèle, ainsi que du système de commande séquentielle des lattes selon un mouvement alternatif.

8. Système selon la revendication 7, comprenant un dispositif de chauffage de l'ensemble de lattes de telle façon à constituer un plancher mouvant chauffant.

9. Installation (1 ; 2 ; 3 ; 4) de traitement en continu de déchets comprenant, en série :
- un premier système (10) de déchiquetage voire de mélange de déchets,
- un deuxième système (21 ; 22) de traitement en continu de déchets par apport thermique pour l'élévation en température des déchets,
- un troisième système (31 ; 32 ; 33) pour le maintien en température des produits, et dans laquelle ledit deuxième système est constitué par un système selon l'une des revendications 1 à 8.

10. Installation (1 ; 2 ; 3 ; 4) de traitement en continu de déchets comprenant, en série :
- un premier système (10) de déchiquetage voire de mélange de déchets,
- un deuxième système (21 ; 22) de traitement en continu de déchets par apport thermique pour l'élévation en température des déchets,
- un troisième système (32) pour le maintien en température des déchets
et dans laquelle ledit troisième système est constitué par un système selon l'une des revendications 1 à 8

11. Utilisation d'un système conforme à l'une des revendications 1 à 8 ou d'une installation selon la revendication 9 ou 10 pour l'hygiénisation de déchets.

12. Utilisation d'un système conforme à l'une des revendications 1 à 8 ou d'une installation selon la revendication 9 ou 10 pour la décontamination de déchets à risque infectieux tels que les déchets médicaux.

## Patentansprüche

1. System (21; 22; 32) zur kontinuierlichen Behandlung von Abfällen durch Wärmezufuhr, zur Hygienisierung von Abfällen oder zur Dekontamination solcher Abfälle mit einem Infektionsrisiko, umfassend:
- eine Umhüllung (210; 220; 320) zur Behandlung der Abfälle;
- eine Vorrichtung (211; 212; 321) zur Wärmezufuhr durch Mikrowellengeneratoren (211a; 221a; 321a), die in Bezug auf die Umhüllung (210; 220; 320) derart angeordnet sind, dass die Mikrowellen in der Umhüllung (210; 220; 320) gehalten werden;
- eine Vorrichtung (213; 223; 323) zum Halten der Produkte und der Dämpfe, die daraus entweichen, im Innen der Umhüllung;
- eine Vorrichtung (212; 222; 322) zum Transportieren durch einen sich bewegenden Boden, der auf dem Grund der Umhüllung (210; 220; 320) liegt, umfassend eine Anordnung von parallel angeordneten Latten, die den Boden bilden, sowie ein Steuersystem der Latten gemäß einer Hinund Herbewegung, das dafür geeignet ist, den Transport von einer Schicht der Abfälle (D) von ihrem Eintritt in die Umhüllung bis zu ihrem Austritt aus der Umhüllung sicherzustellen.

2. System nach Anspruch 1, wobei die Vorrichtung (213; 323) zum Halten der Abfälle und der Dämpfe, die daraus entweichen, eine Halteplatte (213a; 323a) im Inneren der Umhüllung (210; 320) umfasst, die dazu bestimmt ist, auf der Schicht der Abfälle (D) aufzuliegen, die in Bezug auf einen festen Teil des Systems über passive Aufhängungsmittel bewegbar ist, die es der Halteplatte (213a; 323a) gestatten, unter ihrem eigenen Gewicht auf der Schicht der Produkte (D) zu liegen, wobei gleichzeitig ihr Anheben beim Vorschieben der Produkte unter der Einwirkung der Vorrichtung zum Transportieren durch den sich bewegenden Boden ermöglicht wird.

3. System nach Anspruch 2, wobei die Halteplatte (213a; 323a) in der Umhüllung (210; 320) zwischen einerseits den Mikrowellengeneratoren (211a; 321a) der Vorrichtung (211; 321) zur Wärmezufuhr durch Mikrowellengeneratoren und andererseits der Schicht der Abfälle (D) angeordnet ist, wobei die Halteplatte (213a; 323a) dafür bestimmt ist, von den Mikrowellen durchquert zu werden und aus einem für Mikrowellen durchlässigen Material hergestellt ist, wie Polytetrafluorethylen.

4. System nach Anspruch 2 oder 3, wobei die Halteplatte (213a; 323a) an dem festen Teil der Anlage durch ein System von Balken (213b; 323b) aufgehängt ist.

5. System nach Anspruch 1, wobei die Vorrichtung (223) zum Halten der Abfälle und der Dämpfe, die daraus entweichen, ein Förderband im Inneren der Umhüllung (220) umfasst, wobei das Förderband ein nachgiebiges Band (223a) umfasst, das von sich drehenden Führungszylindern (223b) geführt wird, wobei das nachgiebige Band (223a) dafür bestimmt ist, auf die Schicht der Abfälle (D) zu drücken.

6. System nach Anspruch 5, wobei das Förderband in der Umhüllung (220) zwischen einerseits den Mikrowellengeneratoren (221a) der Vorrichtung (221) zur Wärmezufuhr durch Mikrowellengeneratoren und andererseits der Schicht der Abfälle (D) angeordnet ist, wobei das nachgiebige Band (223a) dafür bestimmt ist, von den Mikrowellen durchquert zu werden und aus einem für Mikrowellen durchlässigen Material hergestellt ist, wie Silikon.

7. System nach einem der Ansprüche 1 bis 6, wobei die Vorrichtung (212; 222; 322) zum Transportieren durch einen sich bewegenden Boden aus der Anordnung der Latten, die aus mindestens drei Teilanordnungen (212a, 212b, 212c) bestehen, wobei die Latten parallel angeordnet sind, sowie einem sequentiellen Steuersystem der Latten gemäß einer Hin- und Herbewegung gebildet ist.

8. System nach Anspruch 7, umfassend eine Vorrichtung zum Heizen der Anordnung der Latten, um einen sich bewegenden Heizboden zu bilden.

9. Anlage (1; 2; 3; 4) zur kontinuierlichen Behandlung von Abfällen, umfassend in Serie:
- ein erstes System (10) zum Zerkleinern sogar einer Mischung von Abfällen,
- ein zweites System (21; 22) zur kontinuierlichen Behandlung von Abfällen durch Wärmezufuhr zur Erhöhung der Temperatur der Abfälle,
- ein drittes System (31; 32; 33) zum Halten der Temperatur der Produkte, und wobei das zweite System aus einem System nach einem der Ansprüche 1 bis 8 besteht.

10. Anlage (1; 2; 3; 4) zur kontinuierlichen Behandlung von Abfällen, umfassend in Serie:
- ein erstes System (10) zum Zerkleinern sogar einer Mischung von Abfällen,
- ein zweites System (21; 22) zur kontinuierlichen Behandlung von Abfällen durch Wärmezufuhr zur Erhöhung der Temperatur der Abfälle,
- ein drittes System (32) zum Halten der Temperatur der Abfälle;
und wobei das dritte System aus einem System nach einem der Ansprüche 1 bis 8 besteht.

11. Verwendung eines Systems nach einem der Ansprüche 1 bis 8 oder einer Anlage nach Anspruch 9 oder 10 zur Hygienisierung von Abfällen.

12. Verwendung eines Systems nach einem der Ansprüche 1 bis 8 oder einer Anlage nach Anspruch 9 oder 10 zur Dekontamination von Abfällen mit einem Infektionsrisiko, wie medizinischen Abfällen.

## Claims

1. System (21; 22; 32) for the continuous treatment of waste by thermal input, to make waste hygienic or to decontaminate such waste with a risk of infection comprising:
- a waste treatment chamber (210; 220; 320),
- a device (211; 212; 321) for thermal input by microwave generators (211a; 221a; 321a), arranged with respect to said chamber (210; 220; 320) such that the microwaves are contained in said chamber (210; 220; 320),
- a device (213; 223; 323) for confining products and vapours which are released from it, inside said chamber,
- a moving floor transportation device (212; 222; 322), resting on the bottom of the chamber (210; 220; 320), comprising a set of slats arranged in parallel forming said floor, as well as a system for controlling the slats according to a reciprocating movement, capable of transporting a layer of waste (D) from the entry thereof into the chamber until the exit thereof from the chamber.

2. System according to claim 1, wherein said device (213; 323) for confining waste and vapour which are released from it comprises a confinement plate (213a; 323a) inside said chamber (210; 320), intended to press on the layer of waste (D), mobile with respect to a fixed portion of the system via passive suspension means, making it possible for the confinement plate (213a; 323a) to rest under its own weight on the layer of products (D), while enabling the lifting thereof during the advancement of the products under the action of the moving floor transportation device.

3. System according to claim 2, wherein the confinement plate (213a; 323a) is arranged in said chamber (210; 320) between, on the one hand, microwave generators (211a; 321a) of said device (211; 321) of thermal input by microwave generators and, on the other hand, the layer of waste (D), said confinement plate (213a; 323a) being intended to be passed through by the microwaves and being made of a material which is permeable to microwaves such as polytetrafluoroethylene.

4. System according to claim 2 or 3, wherein said confinement plate (213a; 323a) is suspended to the fixed portion of the installation by a beam system (213b; 323b).

5. System according to claim 1, wherein the device (223) for confining waste and vapours which are released from it comprises a conveyor, inside said chamber (220), said conveyor comprising a flexible band (223a), guided by rotating guiding cylinders (223b), said flexible band (223a) being intended to press on the layer of waste (D).

6. System according to claim 5, wherein the conveyor is arranged in said chamber (220) between, on the one hand, microwave generators (221a) of said device (221) of thermal input by microwave generators and, on the other hand, the layer of waste (D), said flexible band (223a) being intended to be passed through by the microwaves and being made of a material which is permeable to microwaves such as silicone.

7. System according to one of claims 1 to 6, wherein the moving floor transportation device (212; 222; 322) is formed from said set of slats constituted of at least three subsets (212a; 212b; 212c), said slats being arranged in parallel, as well as the system for sequentially controlling the slats according to a reciprocating movement.

8. System according to claim 7, comprising a device for heating the set of slats so as to constitute a heating moving floor.

9. Installation (1; 2; 3; 4) for the continuous treatment of waste comprising, in series:
- a first system (10) for shredding, even mixing waste,
- a second system (21; 22) for the continuous treatment of waste by thermal input for the increase in temperature of waste,
- a third system (31; 32; 33) for maintaining the temperature of the products,
and wherein said second system is constituted by a system according to one of claims 1 to 8.

10. Installation (1; 2; 3; 4) for the continuous treatment of waste comprising, in series:
- a first system (10) for shredding, even mixing waste,
- a second system (21; 22) for the continuous treatment of waste by thermal input for the increase in temperature of waste,
- a third system (32) for maintaining the temperature of the products,
and wherein said third system is constituted by a system according to one of claims 1 to 8.

11. Use of a system according to one of claims 1 to 8 or of an installation according to claim 9 or 10 to make waste hygienic.

12. Use of a system according to one of claims 1 to 8 or of an installation according to claim 9 or 10 to decontaminate waste with a risk of infection such as medical waste.
